# EUROPÄISCHE PATENTANMELDUNG

(11) **EP 4 813 341 A1**
(43) Veröffentlichungstag der Anmeldung: **30.09.2026**
(21) Anmeldenummer: 25165620.3
(22) Anmeldetag: 24.03.2025
(51) Int. Cl.: A61B 90/30, A61B 34/20, A61B 90/20

(54) **MEDIZINISCHES SYSTEM**

(71) Anmelder: B. Braun New Ventures GmbH, 79110 Freiburg im Breisgau (DE)
(72) Erfinder: STAWIASKI, Jean, 79117 Freiburg (DE); DIPPEL, Maximilian, 79115 Freiburg (DE)
(74) Vertreter: Winter, Brandl - Partnerschaft mbB

(57) **Zusammenfassung**

Die vorliegende Offenbarung betrifft ein medizinisches System für einen chirurgischen Eingriff an einem anatomischen Bereich eines Patienten, mit registrierten, radiologischen 3D-Aufnahmedaten des anatomischen Bereiches, einer Erfassungseinheit (2), die eingerichtet ist, eine zeitaktuelle Oberfläche (O) des anatomischen Bereiches zu erfassen, einer Anzeigevorrichtung (6), und einer Steuereinheit (8), die eingerichtet ist, die zeitaktuelle Oberfläche (O) in den radiologischen 3D-Aufnahmedaten auf der Anzeigevorrichtung visuell darzustellen.

## Beschreibung

### Technisches Gebiet

Die vorliegende Offenbarung betrifft ein medizinisches System für einen chirurgischen, insbesondere kranialen/neurochirurgischen, Eingriff an einem anatomischen Bereich eines Patienten.

Aus dem Stand der Technik ist bereits bekannt, einen Patienten mit Hilfe von radiologischen 3D-Aufnahmedaten/Bilddaten und Daten über eine zeitaktuelle Position und/oder Orientierung eines anatomischen Bereichs des Patienten, im Fall des kranialen/neurochirurgischen Eingriffs insbesondere eines Patientenkopfs bzw. eines Patientengesichts, zu registrieren, d.h. die radiologischen 3D-Aufnahmedaten mit der zeitaktuellen Position und/oder Orientierung des anatomischen Bereichs zu verknüpfen, um basierend darauf den chirurgischen Eingriff durch Navigation von Instrumenten, Geräten, etc. gegenüber dem Patienten zu unterstützen. Zur Registrierung können unterschiedliche Methoden angewandt werden, die in der Regel eine äußere Oberfläche des anatomischen Bereichs erfassen und den radiologischen 3D-Aufnahmedaten zuordnen bzw. diese fest miteinander verknüpfen.

Nach einer Öffnung des anatomischen Bereichs des Patienten für den chirurgischen Eingriff kommt es jedoch dazu, dass sich insbesondere innerhalb des Patienten befindliches Gewebe relativ zu der äußeren Oberfläche verlagern kann und die registrierten, radiologischen 3D-Aufnahmedaten zumindest bezüglich des Gewebes nicht mehr der zeitaktuellen Position und/oder Orientierung des anatomischen Bereichs entsprechen.

Insbesondere bei dem kranialen/neurochirurgischen Eingriff verursacht ein Eindringen von Luft sowie eine Resektion eines Teilbereichs des anatomischen Bereichs, dass das Gewebe bzw. eine Hirnmasse kollabiert bzw. zusammenfällt sowie teilweise nicht mehr vorhanden ist. Dieses Phänomen des zusammenfallenden Gewebes ist insbesondere als Gewebeverschiebung ("tissue shift") bzw. als Hirnverschiebung ("brain shift") bekannt.

Aufgrund der Gewebeverschiebung entsteht somit eine Diskrepanz zwischen einer realen/tatsächlichen/zeitaktuellen Position und/oder Orientierung des Gewebes und den registrierten, radiologischen 3D-Aufnahmedaten, so dass die Registrierung der radiologischen 3D-Aufnahmedaten nur noch bezüglich der Position und/oder Orientierung des Schädelknochens gültig ist, aber bezüglich der Position und/oder Orientierung des Gewebes nicht mehr zutreffend ist. Diese Abweichung macht eine präzise Lokalisierung des Gewebes und einer Navigation dazu unmöglich, da aufgrund der Ungenauigkeit ein hohes Risiko zur Verletzung des Gewebes besteht, was gravierende Folgen für den Patienten haben kann.

Im Stand der Technik gibt es bereits Ansätze, um die Navigationsgenauigkeit nach einer solchen Gewebeverschiebung zu korrigieren.

Zum Beispiel ist es aus dem Stand der Technik bekannt, intraoperativ (insbesondere nach der Gewebeverschiebung) eine Ultraschall-Bildgebung einzusetzen, um die Patientenregistrierung lokal zu aktualisieren. Eine solche Ultraschall-Bildgebung ist jedoch zeitintensiv und stört den chirurgischen Ablauf. Zudem können so nur lokale Korrekturen durchgeführt werden.

Auch ist es aus dem Stand der Technik bekannt, intraoperativ (insbesondere nach der Gewebeverschiebung) (neue) radiologische 3D-Aufnahmedaten, etwa MRT-Daten, zu erstellen und einzusetzen, um die für die Registrierung verwendeten Aufnahmedaten zu aktualisieren. Eine erneute Erstellung der radiologischen 3D-Aufnahmedaten ist jedoch zeitintensiv und stört den chirurgischen Ablauf.

Weiter ist es aus dem Stand der Technik bekannt, Augmented Reality einzusetzen, um die Patientenanatomie mit einer präoperativen Aufnahme visuell neu auszurichten. Die Genauigkeit ist jedoch je nach eingesetzten Daten für die Augmented Reality nicht hoch genug.

Ferner ist es aus Stand der Technik bekannt, biomechanische Modelle einzusetzen, um die Registrierung basierend auf aussagekräftigen intraoperativen Messungen, z. B. einer Rekonstruktion der 3D-Oberfläche des Gehirns des Patienten, zu korrigieren. Solche biomechanischen Modelle sind jedoch noch nicht genau genug, so dass insbesondere nicht zwischen der durch Gewebeverschiebung und durch Resektion hervorgerufenen anatomischen Veränderung unterschieden werden kann.

Darüber hinaus ist es aus Stand der Technik bekannt, vorhersagende Modelle der Gewebeverschiebung einzusetzen, um die anatomische Veränderung unter Verwendung von früheren Daten ähnlicher Eingriffe vorherzuzusagen. Solche vorhersagenden Modelle sind jedoch noch nicht genau genug, um den komplexen Prozess der Gewebeverschiebung mit ausreichend hoher Genauigkeit vorhersagen zu können.

Daher liegt der vorliegenden Offenbarung die Aufgabe zugrunde, die Nachteile aus dem Stand der Technik zu vermeiden oder wenigstens zu verringern. Insbesondere soll ein medizinisches System bereitgestellt werden, bei dem die Auswirkungen einer Gewebeverschiebung bezüglich eines navigierten Eingriff reduziert werden, um die Patientensicherheit in ausreichendem Maße sicherstellen zu können.

Die Aufgabe der vorliegenden Offenbarung wird durch ein medizinisches System mit den Merkmalen des Patentanspruchs 1 gelöst. Vorteilhafte Weiterbildungen sind Gegenstand der abhängigen Ansprüche.

Die vorliegende Offenbarung betrifft ein medizinisches System für einen chirurgischen, insbesondere kranialen/neurochirurgischen, Eingriff an einem anatomischen Bereich eines Patienten.

Das medizinische System weist registrierte, radiologische 3D-Aufnahmedaten (3D, dreidimensionale) des anatomischen Bereiches des Patienten auf. Die radiologischen 3D-Aufnahmedaten können vorzugsweise präoperativ (und/oder intraoperativ) erstellt sein. Insbesondere können die radiologischen 3D-Aufnahmedaten MRT-Daten oder CT-Daten sein.

Die radiologischen 3D-Aufnahmedaten sind registriert. Das heißt, dass eine Erfassung von Punkten einer äußeren Oberfläche des Patienten (beispielsweise von Punkten an dem Gesicht des Patienten) als Referenzpunkte und ferner einer Zuordnung dieser Referenzpunkte zu den entsprechenden Punkten der radiologischen 3D-Aufnahmedaten durchgeführt wurde. Dabei stehen verschiedene, hinreichend aus dem Stand der Technik bekannte Registrierungsmethoden zur Verfügung.

Das medizinische System weist eine Erfassungseinheit auf. Die Erfassungseinheit ist eingerichtet, eine zeitaktuelle Oberfläche (d.h. eine Ist-Oberfläche) des anatomischen Bereiches des Patienten zu erfassen. Das heißt, dass die Erfassungseinheit eingerichtet ist, dreidimensionale Oberflächendaten, d.h. mit Tiefeninformationen, der zeitaktuellen Oberfläche zu erstellen. Die Erfassung kann beispielsweise optisch oder taktil erfolgen.

Das medizinische System weist eine Anzeigevorrichtung auf. Die Anzeigevorrichtung kann beispielsweise ein Monitor/Bildschirm sein. Die Anzeigevorrichtung kann zur zweidimensionalen und/oder dreidimensionalen Anzeige eingerichtet sein.

Das medizinische System weist eine Steuereinheit auf. Die Steuereinheit ist eingerichtet, die zeitaktuelle Oberfläche in den radiologischen 3D-Aufnahmedaten auf der Anzeigevorrichtung visuell darzustellen. Das heißt, dass die Oberflächendaten mit den radiologischen 3D-Aufnahmedaten überlagert dargestellt werden, um eine Differenz zwischen den Datensätzen zu visualisieren.

Dies hat den Vorteil, dass dem Operateur zumindest innerhalb der (ursprünglichen) 3D-Aufnahmedaten, die bezüglich der Positionierung der Knochen noch akkurat ist, visualisiert werden kann, inwieweit sich eine von außen erkennbare Oberfläche des Gewebes verändert hat, d.h. durch die Gewebeverschiebung verlagert bzw. durch eine Resektion hinsichtlich ihrer Außenabmessungen bewegt hat. So kann der Operateur diese Veränderung des Gewebes berücksichtigen und gewisse Rückschlüsse auf die Gesamtverlagerung des Gewebes ziehen. Zudem hat die Nutzung der Oberflächendaten den Vorteil, dass diese sehr einfach erzeugt werden können, so dass der Ablauf der Operation nicht gestört wird.

Dabei können die Oberflächendaten und die radiologischen 3D-Aufnahmedaten insbesondere hinsichtlich der Position und/oder Orientierung des Schädelknochens zueinander ausgerichtet werden, da im Bereich des Schädelknochens die Registrierung der radiologischen 3D-Aufnahmedaten noch verwendbar ist. Mit anderen Worten werden die Oberflächendaten nicht beliebig mit den radiologischen 3D-Aufnahmedaten überlagert, sondern in der Weise, dass die registrierten, radiologischen 3D-Aufnahmedaten hinsichtlich der veränderten Gewebeposition so überlagert werden, dass der überlagerten Darstellung entnommen werden kann, wie das Gewebe (bzw. eine Oberfläche des Gewebes) (zeitaktuell) zu den registrierten, radiologischen 3D-Aufnahmedaten des Schädelknochens positioniert ist.

Mit anderen Worten wird gemäß der vorliegenden Offenbarung in dem medizinischen System die zeitaktuelle Oberfläche, beispielsweise während einer Tumor-Resektion, dargestellt. Dazu wird die gemessene/erfasste zeitaktuelle Oberfläche in den radiologischen, insbesondere präoperativ erfassten, 3D-Aufnahmedaten dargestellt, um dem Chirurgen darzustellen, wieviel Gewebe bereits entfernt wurde. Somit kann eine Differenz zwischen der zeitaktuellen Anatomie bzw. Oberfläche des Patienten und den radiologischen 3D-Aufnahmedaten in Echtzeit visualisiert werden. Dabei wird die echte/zeitaktuelle (3D-) Oberfläche der Anatomie des Patienten mit den registrierten, radiologischen, insbesondere präoperativ erfassten, 3D-Aufnahmedaten überlagert.

Gemäß einer bevorzugten Ausführungsform kann die Steuereinheit eingerichtet sein, die Oberfläche in einem 2D-Schnittbild (2D, zweidimensionalen) der radiologischen 3D-Aufnahmedaten, d.h. als eine Überlagerungsdarstellung, auf der Anzeigevorrichtung visuell darzustellen. Insbesondere kann die Oberfläche in dem 2D-Schnittbild als eine Linie visualisiert sein. Das 2D-Schnittbild kann insbesondere ein Sagittalschnitt sein.

Gemäß einer bevorzugten Ausführungsform kann die Steuereinheit eingerichtet sein, basierend auf den 3D-Aufnahmedaten und der zeitaktuellen Oberfläche, eine Gewebeverschiebung eines Kortex (d.h. einer Hirnrinde) zu bestimmen und die Gewebeverschiebung in dem 2D-Schnittbild der radiologischen 3D-Aufnahmedaten auf der Anzeigevorrichtung visuell darzustellen. Insbesondere kann die Steuereinheit eingerichtet sein, (basierend auf den 3D-Aufnahmedaten und der zeitaktuellen Oberfläche) die Gewebeverschiebung einer Oberfläche, beispielsweise einer Außenoberfläche oder einer Innenoberfläche, des Kortex zu bestimmen und zu visualisieren. Insbesondere kann eine Richtung und/oder ein Betrag der Gewebeverschiebung visualisiert sein. Beispielsweise kann die Richtung durch eine Ausrichtung eines Pfeils oder einer Linie visualisiert sein. Beispielsweise kann der Betrag durch eine Länge eines Pfeils oder einer Linie visualisiert sein. So kann ein Anwender die Darstellung auf der Anzeigevorrichtung intuitiv interpretieren.

Gemäß einer bevorzugten Ausführungsform kann die Steuereinheit eingerichtet sein, basierend auf den 3D-Aufnahmedaten und/oder der zeitaktuellen Oberfläche, eine virtuelle Kortexlinie eines resezierten anatomischen Bereichs des Patienten in dem 2D-Schnittbild der radiologischen 3D-Aufnahmedaten auf der Anzeigevorrichtung visuell darzustellen. Der resezierte anatomische Bereich des Patienten ist insbesondere ein Teilbereich des anatomischen Bereichs. Insbesondere in dem Fall, in dem ein Teil des Kortex reseziert wurde, ist die Betrachtung der zeitaktuellen Oberfläche nicht ausreichend, um zwischen der Gewebeverschiebung und einer Resektion zu differenzieren. Die virtuelle Kortexlinie gibt demnach an, an welcher Stelle sich der Kortex befunden hätte, wenn keine Resektion durchgeführt worden wäre. Insbesondere kann die virtuelle Kortexlinie bestimmt werden, indem die (ursprüngliche) Kortexlinie der 3D-Aufnahmedaten, insbesondere auf Basis der (bereits bestimmten) Gewebeverschiebung, verschoben wird oder die durch die Resektion unterbrochene Kortexlinie der zeitaktuellen Oberfläche, insbesondere auf Basis eines bekannten Verlaufs der (ursprünglichen) Kortexlinie der 3D-Aufnahmedaten, vervollständigt wird. So wird der Anwender unterstützt zu erkennen, welche Verlagerungen zwischen den 3D-Aufnahmedaten und der zeitaktuellen Oberfläche jeweils durch die Gewebeverschiebung und jeweils durch die Resektion hervorgerufen worden sind.

Gemäß einer bevorzugten Ausführungsform kann die Erfassungseinheit ein digitales, stereoskopisches Operationsmikroskop sein. Das Operationsmikroskop ist eingerichtet, eine mikroskopische Aufnahme der zeitaktuellen Oberfläche des anatomischen Bereiches zu erstellen. Digital heißt, dass das Operationsmikroskop eingerichtet ist, eine digitale Aufnahme zu erstellen und (insbesondere zur Wiedergabe auf der Anzeigevorrichtung) bereitzustellen. Stereoskopisch heißt, dass das Operationsmikroskop eine (räumliche) Tiefenwahrnehmung hat, bei der eine Tiefeninformation insbesondere aus der Disparität zwischen zwei Aufnahmen, die aus räumlich zueinander versetzten Positionen erstellten sind, generiert wird. Zusätzlich kann zur Rekonstruktion der zeitaktuellen Oberfläche eine stereoskopische Kamera eingesetzt werden. Dabei kann die Rekonstruktion der zeitaktuellen Oberfläche mittels Standard-Photogrammetrie und/oder Ansätze des maschinellen Lernens erfolgen. Vorzugsweise kann das Operationsmikroskop bzw. die stereoskopische Kamera optisch kalibriert sein. So wird sichergestellt, dass die zeitaktuelle Oberfläche präzise erfasst werden kann.

Gemäß einer bevorzugten Ausführungsform kann das medizinische System einen Roboterarm mit einem (endständigen) Endeffektor aufweisen. Das Operationsmikroskop kann den Endeffektor bilden oder an dem Endeffektor angebracht sein. So kann eine Bewegung des Operationsmikroskops im Raum sichergestellt werden.

Gemäß einer bevorzugten Ausführungsform kann das medizinische System ein Navigationssystem aufweisen. Das Navigationssystem ist eingerichtet, das Operationsmikroskop nachzuverfolgen bzw. zu tracken. Das heißt, dass das Navigationssystem eingerichtet ist, eine Position und/oder Orientierung des Operationsmikroskops zu bestimmen und bereitzustellen. Mit anderen Worten ist eine Pose des Operationsmikroskops in dem globalen Koordinatensystem bekannt. So können durch eine Bewegung des Operationsmikroskops bedingte Änderung der mikroskopischen Aufnahme berücksichtigt werden.

Gemäß einer bevorzugten Ausführungsform kann das Navigationssystem einen Patienten-Tracker aufweisen. Der Patienten-Tracker ist eingerichtet, rigide mit dem Patienten verbunden zu werden. Der Patienten-Tracker dient insbesondere zur Referenzierung einer Position und/oder einer Orientierung des Patienten. Dabei kann der Patienten-Tracker (direkt) mit einem Körperteil des Patienten, an welchem der Eingriff stattfinden soll, oder (indirekt) mit einer rigide an dem Körperteil befestigten Vorrichtung (wie einer Patientenbefestigungsvorrichtung) rigide verbunden, so dass (möglichst) keine Relativbewegung auftritt. Bei einem kranialen chirurgischen Eingriff kann der Patienten-Tracker beispielsweise direkt am Kranium oder an einer Kopfklemme, welche den Patienten (an einem Operationstisch) fixiert, befestigt werden.

Gemäß einer bevorzugten Ausführungsform kann die Steuereinheit eingerichtet sein, ein Sichtfeld (auch als Field of View (FoV) bezeichnet) des Operationsmikroskops in den radiologischen 3D-Aufnahmedaten auf der Anzeigevorrichtung visuell darzustellen. Dies hat den Vorteil, dass der Anwender die mikroskopische Aufnahme und die radiologischen 3D-Aufnahmedaten intuitiver miteinander in Verbindung bringen kann.

Gemäß einer bevorzugten Ausführungsform kann die Steuereinheit eingerichtet sein, eine Fokusebene des Operationsmikroskops in den radiologischen 3D-Aufnahmedaten auf der Anzeigevorrichtung visuell darzustellen. Dies hat den Vorteil, dass der Anwender die mikroskopische Aufnahme und die radiologischen 3D-Aufnahmedaten intuitiver miteinander in Verbindung bringen kann.

Es ist zu verstehen, dass mit dem Begriff Fokusebene ein Bereich bezeichnet wird, der typischerweise senkrecht zu einer optischen Achse des Operationsmikroskops ist, in welchem ein sich darin befindendes Objekt scharf darstellbar ist. Dabei kann die Fokusebene linien- oder punktförmig ausgebildet sein. Insbesondere bei dem stereoskopischen Operationsmikroskop resultiert die optische Achse, typischerweise als Mittellinie, aus den einzelnen optischen Achsen. Ebenso resultiert typischerweise die Fokusebene (als Schnitt) aus den einzelnen Fokusebenen. Somit kann ggfls. streng genommen die Fokusebene punkt- oder linienförmig sein, wird aber als Fokusebene bezeichnet. In anderen Worten ist der Begriff Fokusebene funktional und nicht rein geometrisch zu verstehen.

Gemäß einer bevorzugten Ausführungsform kann die Steuereinheit eingerichtet sein, basierend auf der zeitaktuellen Oberfläche und einem bzw. dem Sichtfeld des Operationsmikroskops, einen bzw. den resezierten anatomischen Bereich des Patienten zu bestimmen und, basierend auf den radiologischen 3D-Aufnahmedaten und dem resezierten anatomischen Bereich, ein zeitaktuelles Oberflächenmodell des anatomischen Bereichs des Patienten dreidimensional visualisiert auf der Anzeigevorrichtung darzustellen.

Gemäß einer Weiterbildung kann die Steuereinheit eingerichtet sein, das zeitaktuelle Oberflächenmodell farblich visualisiert auf der Anzeigevorrichtung darzustellen. Dies hat den Vorteil, dass die Darstellung des Oberflächenmodells möglichst realitätsgetreu sein kann und somit leichter für den Anwender mit der realen Situation in Verbindung gebracht werden kann.

Gemäß einer bevorzugten Ausführungsform kann die Steuereinheit eingerichtet sein, das zeitaktuelle Oberflächenmodell mittels direkter Volumendarstellung ("direct volume rendering") zu erstellen. Das heißt, dass das zeitaktuelle Oberflächenmodell erstellt wird, ohne explizit geometrische Oberflächen ("surface rendering") aus den radiologischen 3D-Aufnahmedaten extrahiert werden.

Gemäß einer alternativen bevorzugten Ausführungsform kann die Erfassungseinheit eine Tasteinheit, insbesondere ein Pointer sein. Die Tasteinheit ist eingerichtet, die zeitaktuelle Oberfläche mit einer taktilen Messung abzutasten. So kann die zeitaktuelle Oberfläche erfasst werden.

Gemäß einer alternativen bevorzugten Ausführungsform kann die Erfassungseinheit ein Laserabstandsmesser sein. Der Laserabstandsmesser ist eingerichtet, die zeitaktuelle Oberfläche mit einer optischen Messung zu ermitteln. So kann die zeitaktuelle Oberfläche erfasst werden.

Mit anderen Worten betrifft die vorliegende Offenbarung ein medizinisches System, bei dem eine 3D-Oberfläche der Patientenanatomie mithilfe eines oder mehrerer Bildpaare des stereoskopischen, digitalen Mikroskops erstellt wird. Diese 3D-Oberfläche kann mit Hilfe von Patientenregistrierungsdaten in Bezug auf (präoperative) radiologische Bilddaten des Patienten im Raum platziert werden. Der Schnittpunkt dieser 3D-Oberfläche mit einem bestimmten (präoperativen) radiologischen Bild kann berechnet und dem Chirurgen angezeigt werden. Zudem können auch das Sichtfeld des Mikroskops und seine Fokusebene dargestellt werden. Der Chirurg kann dann die globale Gewebeverschiebung und den resezierten Bereich visuell erkennen. Alternativ oder zusätzlich kann eine rekonstruierte Oberfläche und das Sichtfeld des Mikroskops verwendet werden, um einen Bereich des (präoperativen) radiologischen 3D-Bildes auszuschneiden. Auch die Farbtextur der Oberfläche kann verwendet werden, um das Ergebnis der 3D-Darstellung realistischer zu gestalten. Genauer gesagt entspricht das herausgeschnittene Volumen einem Kegel, dessen Boden der der rekonstruierten Oberfläche entspricht und dessen Spitze der Position des Mikroskops entspricht. Dabei können die Bilddaten des Patienten mit direktem Volumenrendering gerendert werden.

### Kurzbeschreibung der Figuren

Fig. 1 zeigt eine schematische funktionsweise einer Erfassungseinheit eines medizinischen Systems gemäß der vorliegenden Offenbarung,
Fig. 2 zeigt ein 2D-Schnittbild von radiologischen 3D-Aufnahmedaten,
Fig. 3 zeigt eine Visualisierung einer durch die Erfassungseinheit erfassten Oberfläche und einer Gewebeverschiebung in dem medizinischen System gemäß der vorliegenden Offenbarung,
Fig. 4 zeigt eine Visualisierung in dem medizinischen System gemäß der vorliegenden Offenbarung
Fign. 5 und 6 zeigen ein zeitaktuelles Oberflächenmodell gemäß der vorliegenden Offenbarung.

### Beschreibung der Ausführungsbeispiele

Die vorliegende Offenbarung betrifft ein medizinisches System für einen chirurgischen, insbesondere kranialen/neurochirurgischen, Eingriff an einem anatomischen Bereich eines Patienten P, insbesondere an einem Patientenkopf.

Das medizinische System weist eine Erfassungseinheit 2 auf. Die Erfassungseinheit 2 ist eingerichtet, eine zeitaktuelle Oberfläche O (d.h. eine Ist-Oberfläche) des anatomischen Bereiches des Patienten zu erfassen. Das heißt, dass die Erfassungseinheit eingerichtet ist, dreidimensionale Oberflächendaten, d.h. mit Tiefeninformationen, der zeitaktuellen Oberfläche O zu erstellen.

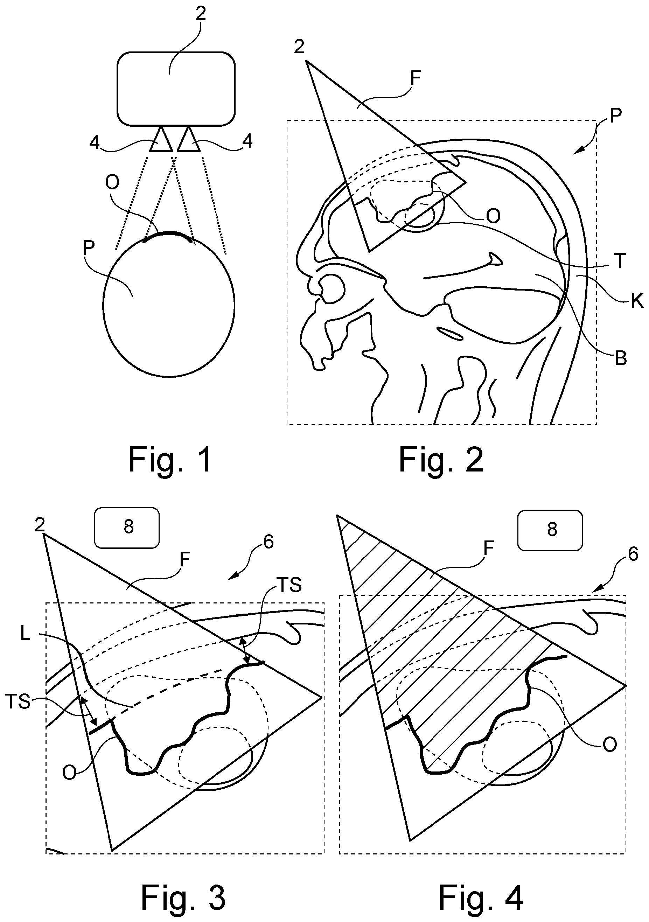

Fig. 1 zeigt einen schematischen Aufbau der Erfassungseinheit 2. Vorzugsweise kann die Erfassungseinheit 2 ein digitales Operationsmikroskop sein. Das Operationsmikroskop ist eingerichtet, eine mikroskopische Aufnahme der zeitaktuellen Oberfläche O des anatomischen Bereiches zu erstellen. Das Operationsmikroskop ist stereoskopisch und weist zwei räumlich zueinander versetzte Kameras 4 auf, um aus einer Disparität zwischen Aufnahmen der zwei Kameras 4 eine Tiefeninformation der zeitaktuellen Oberfläche O zu gewinnen. In Fig. 1 ist jeweils ein Sichtbereich der Kameras 4 eingezeichnet, wobei das Operationsmikroskop in dem überlappenden Sichtbereich der Kameras 4 die zeitaktuelle Oberfläche O dreidimensional erfasst.

Vorzugsweise kann die Erfassungseinheit 2 durch ein Navigationssystem nachverfolgt bzw. getrackt sein. Das heißt, dass eine Position und/oder Orientierung der Erfassungseinheit 2 (auch bei Bewegung der Erfassungseinheit 2) bekannt ist.

Das medizinische System weist radiologische 3D-Aufnahmedaten (3D, dreidimensionale) des anatomischen Bereiches des Patienten P auf. Die radiologischen 3D-Aufnahmedaten können vorzugsweise präoperativ (und/oder intraoperativ) erstellt sein. Insbesondere können die radiologischen 3D-Aufnahmedaten MRT-Daten oder CT-Daten sein.

Fig. 2 zeigt solche radiologische 3D-Aufnahmedaten. Die radiologischen 3D-Aufnahmedaten sind registriert, d.h. mit realen Positionsdaten des Patienten verknüpft. In den radiologischen 3D-Aufnahmedaten ist insbesondere ein Schädelknochen K zu erkennen sowie eine innerhalb des Schädelknochens K befindlichen Hirnmasse B. Zudem ist ein Tumor T zu erkennen. In Fig. 2 ist lediglich ein 2D-Schnittbild (hier ein Sagittalschnitt) der 3D-Aufnahmedaten gezeigt.

Eine Position und Orientierung der Erfassungseinheit 2 bzw. des Operationsmikroskops ist in Fig. 2 durch ein Sichtfeld F des Operationsmikroskops und die durch das Operationsmikroskop erfasste Oberfläche O angedeutet. In Fig. 2 ist zu erkennen, dass der Schädelknochen K bereits geöffnet ist und somit eine Teiloberfläche der Hirnmasse B von außen erkennbar ist bzw. durch die Erfassungseinheit 2 erfassbar ist. Dabei wurde ein Teil des Tumors T bereits reseziert.

Das medizinische System weist eine Anzeigevorrichtung 6 auf. Die Anzeigevorrichtung 6 bzw. die auf der Anzeigevorrichtung 6 dargestellten Inhalte sind in Fign. 3 bis 6 gezeigt. Die Anzeigevorrichtung 6 kann beispielsweise ein Monitor/Bildschirm sein. Die Anzeigevorrichtung 6 kann zur zweidimensionalen Anzeige und/oder zur dreidimensionalen Anzeige eingerichtet sein.

Das medizinische System weist eine Steuereinheit 8 auf. Die Steuereinheit 8 ist eingerichtet, die zeitaktuelle Oberfläche O in den radiologischen 3D-Aufnahmedaten auf der Anzeigevorrichtung 6 visuell darzustellen. Das heißt, dass die Oberflächendaten mit den radiologischen 3D-Aufnahmedaten überlagert dargestellt werden, um eine Differenz zwischen den Datensätzen zu visualisieren.

In Fig. 3 ist dargestellt, dass die zeitaktuelle Oberfläche O in dem 2D-Schnittbild der radiologischen 3D-Aufnahmedaten, d.h. als eine Überlagerungsdarstellung, auf der Anzeigevorrichtung 6 visuell dargestellt ist. Insbesondere kann die Oberfläche O in dem 2D-Schnittbild als eine Linie visualisiert sein. Das heißt, dass die Linie den Verlauf der Oberfläche O in der auf dem 2D-Schnittbild abgebildeten Ebene darstellt.

Wie in Fig. 3 zudem zu erkennen ist, kann basierend auf den 3D-Aufnahmedaten und der zeitaktuellen Oberfläche O eine Gewebeverschiebung TS eines Kortex (d.h. einer Hirnrinde) in dem 2D-Schnittbild der radiologischen 3D-Aufnahmedaten auf der Anzeigevorrichtung 6 visuell dargestellt werden. In Fig. 3 ist die Gewebeverschiebung TS durch Pfeile angedeutet, die sich von einem bestimmten Punkt der Kortex in den 3D-Aufnahmedaten zu dem verlagerten bestimmten Punkt der Kortex auf der zeitaktuellen Oberfläche O erstrecken. Dabei kann insbesondere eine Richtung der Gewebeverschiebung TS durch eine Ausrichtung eines Pfeils visualisiert sein. Dabei kann ein Betrag der Gewebeverschiebung TS durch eine Länge des Pfeils visualisiert sein.

Wie in Fig. 3 zudem zu erkennen ist, kann basierend auf den 3D-Aufnahmedaten und der zeitaktuellen Oberfläche O eine virtuelle Kortexlinie L eines resezierten anatomischen Bereichs des Patienten P in dem 2D-Schnittbild der radiologischen 3D-Aufnahmedaten auf der Anzeigevorrichtung 6 visuell dargestellt werden. In Fig. 3 ist die virtuelle Kortexlinie L gestrichelt angedeutet. Dabei gibt die virtuelle Kortexlinie an, an welcher Stelle sich der Kortex befunden hätte, wenn keine Resektion durchgeführt worden wäre.

Wie in Fig. 3 zudem zu erkennen ist, kann das Sichtfeld F des Operationsmikroskops in dem 2D-Schnittbild der radiologischen 3D-Aufnahmedaten auf der Anzeigevorrichtung 6 visuell dargestellt werden. In Fig. 3 ist das Sichtfeld F als ein von der Erfassungseinheit 2 bzw. dem Operationsmikroskop ausgehender Sichtkegel angedeutet. Um das Sichtfeld F darstellen zu können, muss eine Position und/oder Orientierung des Operationsmikroskops bekannt sein, was durch das Navigationssystem sichergestellt wird.

Zudem kann eine Fokusebene des Operationsmikroskops in den radiologischen 3D-Aufnahmedaten auf der Anzeigevorrichtung visuell dargestellt werden, auch wenn dies nicht explizit dargestellt ist.

Wie in Fig. 4 zu erkennen ist, kann ein fehlender anatomischer Bereich des Patienten P, welcher innerhalb des Sichtfelds F des Operationsmikroskops liegt, in dem 2D-Schnittbild der radiologischen 3D-Aufnahmedaten auf der Anzeigevorrichtung 6 visuell dargestellt werden. Beispielsweise kann der fehlende anatomische Bereich schraffiert angedeutet werden. Der fehlende anatomische Bereich kann insbesondere aus der Öffnung des Schädelknochens K, der Gewebeverschiebung TS sowie der Resektion von Gewebe resultieren.

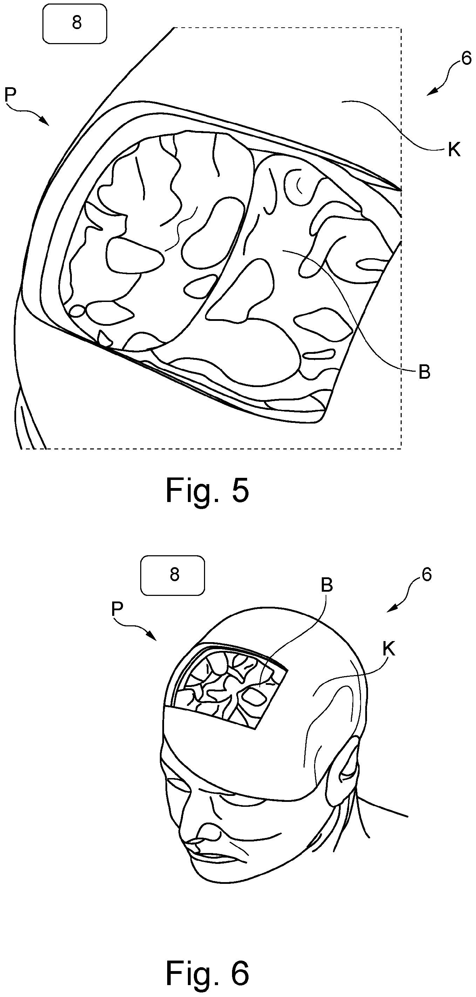

Gemäß einer weiteren bevorzugten Ausführungsform kann die Steuereinheit 8 eingerichtet sein, basierend auf der zeitaktuellen Oberfläche O und einem bzw. dem Sichtfeld F des Operationsmikroskops, einen bzw. den resezierten anatomischen Bereich des Patienten P zu bestimmen und, basierend auf den radiologischen 3D-Aufnahmedaten und dem resezierten anatomischen Bereich, ein zeitaktuelles Oberflächenmodell des anatomischen Bereichs des Patienten P dreidimensional visualisiert auf der Anzeigevorrichtung 6 darzustellen. Ein solches Oberflächenmodell ist insbesondere in Fign. 5 und 6 dargestellt.

Vorzugsweise kann das zeitaktuelle Oberflächenmodell farblich visualisiert auf der Anzeigevorrichtung 6 darzustellen. Dabei kann das zeitaktuelle Oberflächenmodell mittels direkter Volumendarstellung ("direct volume rendering") erstellt sein.

## Patentansprüche

1. Medizinisches System für einen chirurgischen Eingriff an einem anatomischen Bereich eines Patienten, mit
registrierten, radiologischen 3D-Aufnahmedaten des anatomischen Bereiches, einer Erfassungseinheit (2), die eingerichtet ist, eine zeitaktuelle Oberfläche (O) des anatomischen Bereiches zu erfassen,
einer Anzeigevorrichtung (6), und
einer Steuereinheit (8), die eingerichtet ist, die zeitaktuelle Oberfläche (O) in den radiologischen 3D-Aufnahmedaten auf der Anzeigevorrichtung (6) visuell darzustellen.

2. Medizinisches System nach Anspruch 1, **dadurch gekennzeichnet, dass** die Steuereinheit (8) eingerichtet ist, die Oberfläche in einem 2D-Schnittbild der radiologischen 3D-Aufnahmedaten auf der Anzeigevorrichtung (6) visuell, insbesondere als eine Linie, darzustellen.

3. Medizinisches System nach Anspruch 1 oder 2, **dadurch gekennzeichnet, dass** die Steuereinheit (8) eingerichtet ist, basierend auf den radiologischen 3D-Aufnahmedaten und der zeitaktuellen Oberfläche (O), eine Gewebeverschiebung (TS) eines Kortex zu bestimmen und die Gewebeverschiebung (TS) in dem 2D-Schnittbild der radiologischen 3D-Aufnahmedaten auf der Anzeigevorrichtung (6) visuell darzustellen.

4. Medizinisches System nach einem der Ansprüche 1 bis 3, **dadurch gekennzeichnet, dass** die Steuereinheit (8) eingerichtet ist, basierend auf den radiologischen 3D-Aufnahmedaten und der zeitaktuellen Oberfläche (O), eine virtuelle Kortexlinie (L) eines resezierten anatomischen Bereichs des Patienten (P) in dem 2D-Schnittbild der radiologischen 3D-Aufnahmedaten auf der Anzeigevorrichtung (6) visuell darzustellen.

5. Medizinisches System nach einem der Ansprüche 1 bis 4, **dadurch gekennzeichnet, dass** die Erfassungseinheit (2) ein digitales, stereoskopisches Operationsmikroskop ist, das eingerichtet ist, eine mikroskopische Aufnahme der zeitaktuellen Oberfläche (O) des anatomischen Bereiches zu erstellen.

6. Medizinisches System nach Anspruch 5, **dadurch gekennzeichnet, dass** das medizinische System ein Navigationssystem aufweist, das eingerichtet ist, das Operationsmikroskop nachzuverfolgen, und die Steuereinheit (8) eingerichtet ist, ein Sichtfeld (F) des Operationsmikroskops in den radiologischen 3D-Aufnahmedaten auf der Anzeigevorrichtung (6) visuell darzustellen.

7. Medizinisches System nach Anspruch 5 oder 6, **dadurch gekennzeichnet, dass** die Steuereinheit (8) eingerichtet ist, eine Fokusebene des Operationsmikroskops in den radiologischen 3D-Aufnahmedaten auf der Anzeigevorrichtung (6) visuell darzustellen.

8. Medizinisches System nach einem der Ansprüche 5 bis 7, **dadurch gekennzeichnet, dass** die Steuereinheit (8) eingerichtet ist, basierend auf der zeitaktuellen Oberfläche (O) und einem Sichtfeld (F) des Operationsmikroskops, einen resezierten anatomischen Bereich des Patienten(P) zu bestimmen und, basierend auf den radiologischen 3D-Aufnahmedaten und dem resezierten anatomischen Bereich, ein zeitaktuelles Oberflächenmodell des anatomischen Bereichs des Patienten (P) dreidimensional visualisiert auf der Anzeigevorrichtung (6) darzustellen.

9. Medizinisches System nach Anspruch 8, **dadurch gekennzeichnet, dass** die Steuereinheit (8) eingerichtet ist, das zeitaktuelle Oberflächenmodell mittels direkter Volumendarstellung zu erstellen.

10. Medizinisches System nach einem der Ansprüche 1 bis 4, **dadurch gekennzeichnet, dass** die Erfassungseinheit (2) eine Tasteinheit ist, die eingerichtet ist, die zeitaktuelle Oberfläche mit einer taktilen Messung abzutasten und/oder dass die Erfassungseinheit (2) ein Laserabstandsmesser ist, der eingerichtet ist, die zeitaktuelle Oberfläche mit einer optischen Messung zu ermitteln.
